(19) Europäisches Patentamt
European Patent Office
Office européen des brevets

(11) **EP 3 227 427 B1**

(12) **FASCICULE DE BREVET EUROPEEN**

(45) Date de publication et mention
de la délivrance du brevet:
**21.08.2019 Bulletin 2019/34**

(21) Numéro de dépôt: **15810693.0**

(22) Date de dépôt: **02.12.2015**

(51) Int Cl.:
*C12M 1/00* (2006.01)

(86) Numéro de dépôt international:
**PCT/FR2015/053294**

(87) Numéro de publication internationale:
**WO 2016/087779 (09.06.2016 Gazette 2016/23)**

(54) **DISPOSITIF DE PRODUCTION D'UNE CULTURE PHOTOSYNTHÉTIQUE AU MOYEN D'UN PHOTO-BIORÉACTEUR ET D'AU MOINS UN DISTRIBUTEUR DE LUMIÈRE**

VORRICHTUNG ZUR HERSTELLUNG EINER PHOTOSYNTHETISCHEN KULTUR MITTELS EINES LICHTBIOREAKTORS UND MINDESTENS EINEM LICHTVERTEILER

DEVICE FOR PRODUCING A PHOTOSYNTHETIC CULTURE BY MEANS OF A PHOTO-BIOREACTOR AND AT LEAST ONE LIGHT DISTRIBUTOR

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **02.12.2014 FR 1461778**

(43) Date de publication de la demande:
**11.10.2017 Bulletin 2017/41**

(73) Titulaire: **SUNOLEO**
**84120 Pertuis (FR)**

(72) Inventeur: **BARBARIN, Frédéric**
**36290 Obterre (FR)**

(74) Mandataire: **Jacobacci Coralis Harle**
**32, rue de l'Arcade**
**75008 Paris (FR)**

(56) Documents cités:
**EP-A1- 0 112 556          WO-A1-2012/152637**
**WO-A1-2013/011448     US-A- 4 900 678**

• **DATABASE WPI Week 200058 Thomson Scientific, London, GB; AN 2000-605213 XP002745063, & JP 2000 228973 A (SUMITOMO HEAVY IND LTD) 22 août 2000 (2000-08-22)**

EP 3 227 427 B1

**Description**

## DOMAINE TECHNIQUE DE L'INVENTION

L'invention se rapporte au domaine des dispositifs de production de culture photosynthétique, et notamment au moyen d'un photo-bioréacteur.

## ETAT DE LA TECHNIQUE ANTERIEURE

La culture de micro-algues est au coeur de procédés de valorisation. En effet, les micro-algues sont une source alternative notamment pour la production de biocarburant, de biomasse, d'huiles, de protéines, d'esters ou d'éthanol. En outre, de telles cultures peuvent être utilisées pour la valorisation et la purification des eaux usées, le traitement des rejets de fertilisants, $CO_2$, $NO_x$, $SO_x$, de certaines usines.

Le développement des cultures photosynthétiques de micro-algues nécessite cependant la mise en place de systèmes de production spécifiques, à adapter selon l'application visée, les conditions industrielles d'exploitation, la souche à cultiver, les conditions géographiques, climatiques ou d'ensoleillement.

En outre, une difficulté bien connue dans ce domaine concerne la pénétration de la lumière dans le photo-bioréacteur. En effet, sur une surface de terrain donnée, une simple étendue d'eau horizontale, comme les « raceways » (photo-bioréacteur à grand bassin ouvert, de faible profondeur), n'utilise que partiellement le potentiel solaire puisque, dès quelques centimètres de profondeur, l'opacité de l'eau selon sa concentration en micro-algues, limite la pénétration de la lumière en profondeur. Le surplus de lumière directe ne pouvant traverser l'opacité des milieux de culture (ou eau algale) est alors absorbé partiellement par celle-ci sous forme de chaleur, au lieu de servir à la croissance des micro-algues, entraînant un problème d'évaporation de l'eau.

En outre, les « raceways » peuvent générer des problèmes liés à des contaminations extérieures (bactériens, prédateurs, algues concurrentes introduites...) qui viennent nuire à la production. Un autre inconvénient des « raceways » concerne le taux élevé d'évaporation de l'eau.

Pour pallier ces problèmes, des photo-bioréacteurs à fines couches étanches ont été développés. Cependant, un système de refroidissement est indispensable pour éviter une altération des micro-algues, rendant la gestion de la culture plus compliquée et diminuant son rendement. En effet, ces photo- bioréacteurs requièrent des équipements de gestion complexe proportionnellement au volume d'eau exploitée, ce qui implique que leur industrialisation est impossible pour la production de masse.

Un autre mode de réalisation a été développé dans lequel, les photo-bioréacteurs utilisent des diffuseurs de lumière en leur sein afin d'augmenter le volume d'eau utile pour la production de micro-algues.

De tels dispositifs sont décrits dans la demande internationale WO2013/063075, dans laquelle, des LEDs ont été disposées à l'intérieur du photo-bioréacteur afin d'augmenter le rendement de la culture photosynthétique.

En outre, certains des bioréacteurs de l'art antérieur ont une construction relativement complexe et utilisent parfois des optiques complexes pour éclairer l'eau algale.

Ce type de dispositif nécessite une alimentation en énergie augmentant le coût de revient de la culture de micro-algues. Par conséquent, la lumière solaire est souvent préférée.

Le document WO 2012/152637 divulgue un photobioréacteur, dans lequel un élément diffuseur de lumière placé dans l'enceinte de culture est couplé optiquement à une source lumineuse artificielle, par exemple une ou plusieurs LED. En outre ce dispositif n'est pas adapté au bassin de culture de grande taille, dans la mesure où les diffuseurs de lumières qui ne sont pas remplis de fluide ne peuvent résister à une pression hydrostatique de l'eau trop importante.

La demande internationale WO 2009/116853 présente une alternative de diffuseurs de lumière, en l'occurrence de lumière solaire par l'ajout dans la cuve d'un dispositif rigide transparent comprenant au moins une surface de réception de lumière agencée pour recevoir la lumière solaire et au moins une surface d'émission, immergée dans l'eau algale, agencée pour émettre au moins une partie de la lumière reçue dans l'eau algale.

Ce type de photo-bioréacteur outre d'utiliser des matériaux aux parois épaisses et coûteuses, nécessite une exposition importante au rayonnement solaire, ce qui accroît l'évaporation de l'eau algale et rend plus compliquée la gestion de la culture photosynthétique.

En outre, de tels diffuseurs de lumière présentent un encombrement spatial très important quel que soit le type de cuves utilisées. Ceci rend difficile leur maintenance, leurs remplacements et leurs utilisations dans des photo-bioréacteurs de taille industrielle.

La demande internationale WO 2013/011448 décrit un photo-bioréacteur dans lequel les distributeurs de lumières composés de manches réalisées en un matériau souple, transparent, résistant à la traction et la pression, sont suspendues verticale à un support et immergées dans la solution algale. Le support ferme de préférence le bassin. Ce support doit donc présenter la résistance mécanique nécessaire pour supporter le poids de l'ensemble des manches remplies d'eau, ce qui limite la taille des bassins.

## EXPOSE DE L'INVENTION

L'invention vise à remédier aux inconvénients de l'état de la technique et notamment à proposer un dispositif de production d'une culture photosynthétique comprenant au moins un photo-bioréacteur formant une enceinte munie de moyens d'alimentation/évacuation et comprenant :

- un liquide aqueux comprenant une culture photosynthétique,
- au moins un moyen d'alimentation et d'évacuation en fluides de ladite enceinte coopérant avec un système de gestion,
- au moins un distributeur de lumière comprenant :

  - au moins une première paroi agencée pour recevoir de la lumière à une extrémité proximale,
  - au moins une seconde paroi agencée pour émettre au moins une partie de la lumière reçue,
  - une cavité étanche délimitée par lesdites au moins une première et une seconde parois,
  - au moins une partie de la paroi émettrice est immergée dans le liquide aqueux comprenant la culture photosynthétique,
  - au moins un fluide remplissant au moins en partie ladite cavité étanche,

- un moyen de recouvrement dudit photo-bioréacteur apte à limiter l'évaporation dudit liquide aqueux,

caractérisé en ce que ledit moyen de recouvrement est muni d'au moins une ouverture, apte à maintenir ledit au moins un distributeur de lumière dans une position fixe dans ladite enceinte du photo-bioréacteur caractérisé en ce que ledit au moins un distributeur de lumière est flottant et permet de soutenir le moyen de recouvrement au-dessus du plan d'eau algale.

Par « liquide aqueux comprenant une culture photosynthétique », on entend de préférence toutes cultures de micro-algues ; les micro-algues sont choisies en fonction de l'application visée (par exemple la production de protéines, huile, éthanol, biomasse) et des conditions de culture, notamment géographiques, climatiques et de températures. En outre, le terme « liquide aqueux comprenant une culture photosynthétique » peut être remplacé par « eau algale » sans que cela influe sur la portée de la protection accordée par la présente demande.

Par « fluide », on entend, tout liquide ou gaz approprié pour le fonctionnement du photo-bioréacteur ou le développement de la culture photosynthétique.

Par « distributeur de lumière », on entend, tout corps possédant au moins deux parois, la première recevant un rayonnement lumineux, de préférence solaire, la seconde émettrice d'au moins une partie du rayonnement lumineux. De préférence, ledit corps est un cylindre ou une variation d'un cylindre s'étendant pour sa hauteur, selon un axe X orthogonal au plan support sur lequel repose ladite enceinte dudit photo-bioréacteur. En outre, la hauteur dudit au moins un distributeur de lumière est proche de la hauteur de ladite enceinte. C'est-à-dire que la hauteur dudit au moins un distributeur de lumière est comprise entre 100% et 85% de la hauteur de ladite enceinte.

Par « extrémité proximale », on entend l'extrémité la plus éloignée du plan support pour une utilisation normale dudit au moins un distributeur de lumière, c'est-à-dire lorsque celui-ci est immergé dans le photo-bioréacteur d'axe X.

Par « extrémité distale », on entend l'extrémité la plus proche du plan support pour une utilisation normale dudit au moins un distributeur de lumière

Par « moyen de recouvrement », on entend, tout moyen permettant de fermer la surface supérieure dudit photo-bioréacteur afin de former une enceinte étanche, de préférence hermétique, par exemple, un couvercle, une bâche, ou tout autre moyen techniquement équivalent. En outre le moyen de recouvrement peut être transparent ou opaque, coloré, et il peut être réalisé à partir d'une bande en polymère découpée et soudée, ou d'une seule pièce.

Avantageusement, une coopération de forme entre au moins une bordure de ladite ouverture dudit moyen de recouvrement et un relief de surface dudit au moins un distributeur de lumière est réalisée, afin de maintenir ledit au moins un distributeur de lumière dans une position fixe dans ladite enceinte du photo-bioréacteur.

Préférentiellement, ladite paroi de réception de lumière dudit au moins un distributeur de lumière est disposée à l'extérieur du volume défini par ladite enceinte du photo-bioréacteur.

En d'autres termes, au moins une partie dudit au moins un distributeur de lumière n'est pas recouverte par le moyen de recouvrement et dépasse dudit moyen de recouvrement, de manière à ce que le plan formé par ledit moyen de recouvrement sépare lesdites au moins une première et une seconde parois dudit au moins un distributeur de lumière.

Avantageusement, le nombre ($nb_{distributeur}$), la hauteur immergée ($H_{distributeur}$) dudit au moins un distributeur de lumière et l'espacement (d) entre les différents distributeurs de lumière sont déterminés à partir des formules suivantes: Dans une enceinte de section transversale circulaire ou rectangulaire de surface $S_{enceinte}$(en m$^2$),

$$nb_{distributeur} = \frac{4.\xi.S_{enceinte}}{\pi.D^2} \qquad (1)$$

$\xi$: la fraction volumique occupée par les distributeurs de lumière, rapportée au volume total d'eau dans l'enceinte.

La fraction $\xi$ est de préférence comprise entre 0,6 à 0,8.

**D** : le diamètre du distributeur de lumière (en m).

Cette précédente équation ne tient pas compte des effets de bord.

$$H_{distributeur} = \frac{D}{4.q_2}(\eta.q_{solaire} - q_2) \qquad (2)$$

**$H_{distributeur}$**: Hauteur de la seconde paroi du distributeur de lumière (partie immergée) (en mm)

$q_{solaire}$ : flux incident d'une journée ensoleillée (en $\mu$moles de photons/m$^2$.s$^{-1}$).

$q_2$: flux lumineux souhaité à la paroi émettrice de lumière du distributeur de lumière pour la bonne production d'une souche de micro-algue donnée (en $\mu$mol/m$^2$.s$^{-1}$).

$\eta$ : le rendement de transmission entre la première paroi réceptrice de lumière et la seconde paroi émettrice du distributeur de lumière.

La hauteur **$H_{distributeur}$** peut être comprise entre 1m et 12m, de préférence entre 4m et 8m, alors que le diamètre du distributeur de lumière peut être compris entre 1m et 3m.

$$d = \sqrt{\frac{\pi.D^2}{4.\xi.\cos 30}} \qquad (3)$$

**d** : distance en mètre entre les axes longitudinaux X de 2 distributeurs de lumière.

De préférence, ledit au moins un distributeur de lumière est muni de moyens d'alimentation et d'extraction en fluides. Lesdits moyens d'alimentation et d'extraction en fluides pouvant être indépendants, partiellement ou totalement confondus les uns des autres.

De préférence ledit au moins un fluide remplissant au moins en partie ladite cavité étanche est composé d'eau et d'air. Ainsi, l'immersion dudit au moins un distributeur de lumière dans l'eau algale est contrôlée par le ratio eau/air présent dans ladite cavité dudit au moins un distributeur de lumière.

En outre, ledit fluide remplissant au moins en partie ladite cavité étanche est apte à mettre en pression le volume intérieur dudit au moins un distributeur de lumière de façon à rendre étanche la coopération de forme entre la bordure de ladite ouverture et ledit relief de surface dudit au moins un distributeur de lumière.

Par ailleurs, lesdits distributeurs de lumière sont flottants et permettent de soutenir le moyen de recouvrement au-dessus du plan d'eau algale, avantageusement à une distance comprise entre 10cm et 50cm. Ainsi, dans un tel mode de réalisation de l'invention, les distributeurs de lumière font office de flotteurs maintenant le moyen de recouvrement au-dessus du plan d'eau algale. Le maintien du moyen de recouvrement au-dessus du plan d'eau algale autorise la circulation des gaz entre la surface de l'eau algale et le moyen de recouvrement. Une telle réalisation du dispositif de l'invention permet de s'affranchir de l'utilisation d'un support rigide au-dessus du plan d'eau algale et par conséquent du problème de portance. Il est ainsi possible d'utiliser, sans limitation, un bassin de culture de grande taille. Ainsi le moyen de recouvrement peut être une structure non rigide comme une simple bâche.

Avantageusement, ledit au moins un distributeur de lumière est muni d'un moyen d'ancrage disposé à une extrémité distale.

Par « moyen d'ancrage », on entend toute masse attachée ou non au fond du photo-bioréacteur, tout moyen de liaison entre ledit au moins un distributeur de lumière et le fond du photo-bioréacteur tel qu'une corde, une chaîne, ou tout autre moyen connu de l'homme du métier, apte à immobiliser dans une position souhaitée ledit au moins un distributeur de lumière.

Selon une autre caractéristique, lesdites parois dudit au moins un distributeur de lumière sont formées par au moins une membrane souple avec de préférence, un haut degré de transparence et une résistance à l'élasticité. Une telle membrane souple peut être en polymère, par exemple de polyéthylène, du polypropylène ou du polyamide, ou du PVC, ou encore une multicouche de polymères ayant des propriétés qui se complètent.

Ainsi, selon cette caractéristique, ledit au moins un distributeur de lumière est gonflable/dégonflable. C'est-à-dire que pour obtenir sa forme d'utilisation, ledit au moins un distributeur de lumière doit avoir ladite cavité étanche remplie de fluide, notamment d'eau et d'air, et avoir une pression supérieure à la pression atmosphérique, de préférence environ à 50 hectopascals.

Par ailleurs, le polymère de la membrane peut présenter un certain degré d'étanchéité à l'oxygène, sur toute sa surface ou localement, afin de limiter la porosité des parois du diffuseur de lumière. Suivant le perfectionnement de la gestion de l'air dans la partie proximale du diffuseur de lumière, plusieurs voies peuvent être retenues. Soit de chercher à limiter au maximum les échanges gazeux avec l'environnement extérieur afin de diminuer l'apport en air pour maintenir à son niveau d'immersion le diffuseur de lumière. Dans cette solution, la porosité à l'oxygène pourrait être par exemple inférieure à $0,1 \text{ cm}^3/\text{m}^2.\text{jour}^{-1}$. Soit au contraire, de faciliter l'évacuation de l'oxygène de l'eau algale vers l'eau du diffuseur de lumière.

En outre, lorsque ladite cavité étanche dudit au moins un distributeur de lumière ne contient pas de fluide, notamment lorsqu'il est stocké ou non encore installé dans ledit photo-bioréacteur, ledit au moins un distributeur de lumière peut être plié, roulé afin de limiter son encombrement spatial.

Avantageusement, ladite membrane en polymère a reçu ou comprend lors de sa fabrication au moins un traitement de surface intérieur et/ou extérieur parmi les suivants : anti-goutte, anti-poussière, filtre UV et/ou infrarouges, stabilisants UV, effet diffusant, semi réfléchissant, hydrophobe, anti-poussière, anti-algue (par exemple : oxydes de titane).

Par exemple, un traitement aux oxydes de titane permet d'éviter sur la première paroi, réceptrice de la lumière solaire, la formation de gouttes de condensation susceptibles de perturber le passage de la lumière dans ledit au moins un distributeur de lumière et sur la seconde paroi d'éviter le phénomène de « fouling », c'est-à-dire le dépôt d'algues sur les parois.

Un traitement sur la surface intérieure avec un filtre infrarouge long permet de garder l'infrarouge long dans le distributeur de lumière afin de chauffer l'intérieur par effet de serre.

Un traitement anti-UV permet d'éviter la destruction de la culture photosynthétique par les UV.

Un traitement semi réfléchissant permet un meilleur acheminement de la lumière vers la partie distale du distributeur de lumière par un « effet miroir », dont l'absorption lumineuse intrinsèque ne devra pas dépasser 10%. Ce traitement local est situé du côté proximal sur la seconde paroi à l'intérieur de la cavité étanche du distributeur de lumière, et peut être réalisé par trempage ou par pulvérisation d'un matériau métallique.

Selon une autre caractéristique, ledit photo-bioréacteur comprend au moins un moyen de bullage avec apport en $CO_2$, disposé au moins sur une partie du fond de l'enceinte du photo-bioréacteur, de façon à permettre un brassage permanent dudit liquide aqueux comprenant ladite culture photosynthétique.

Par « moyen de bullage », on entend tout moyen connu de l'homme du métier apte à apporter au sein de ladite enceinte du gaz, notamment sous forme de bulles.

## BREVE DESCRIPTION DES FIGURES

D'autres caractéristiques, détails et avantages de l'invention ressortiront à la lecture de la description qui suit, en référence aux figures annexées, qui illustrent :

- la figure 1, dévoile une représentation 3D d'un mode de réalisation selon l'invention,
- la figure 2, dévoile une représentation 3D d'un mode de réalisation du photo-bioréacteur sans le moyen de recouvrement,
- la figure 3 est une vue détaillée d'un mode de réalisation d'un distributeur de lumière installé dans un photo-bioréacteur selon l'invention, maintenu dans une position fixe par le moyen de recouvrement,
- la figure 4 est une coupe longitudinale d'un mode de réalisation d'un distributeur de lumière,
- la figure 5 est une vue du dessus d'un mode de réalisation d'une partie du moyen de recouvrement,
- la figure 6 est un schéma de l'extrémité proximale d'un mode de réalisation dudit distributeur de lumière,
- la figure 7 est une coupe schématique orthogonale au plan formé par le support d'un mode de réalisation du réseau d'alimentation et d'extraction/refoulement des fluides du photo-bioréacteur,
- la figure 8 dévoile une représentation en trois dimensions d'un mode de réalisation à grande échelle d'un dispositif de production selon l'invention comprenant plusieurs photo-bioréacteurs.

Pour plus de clarté, les éléments identiques ou similaires sont repérés par des signes de référence identiques sur l'ensemble des figures.

## DESCRIPTION DETAILLEE D'UN MODE DE REALISATION

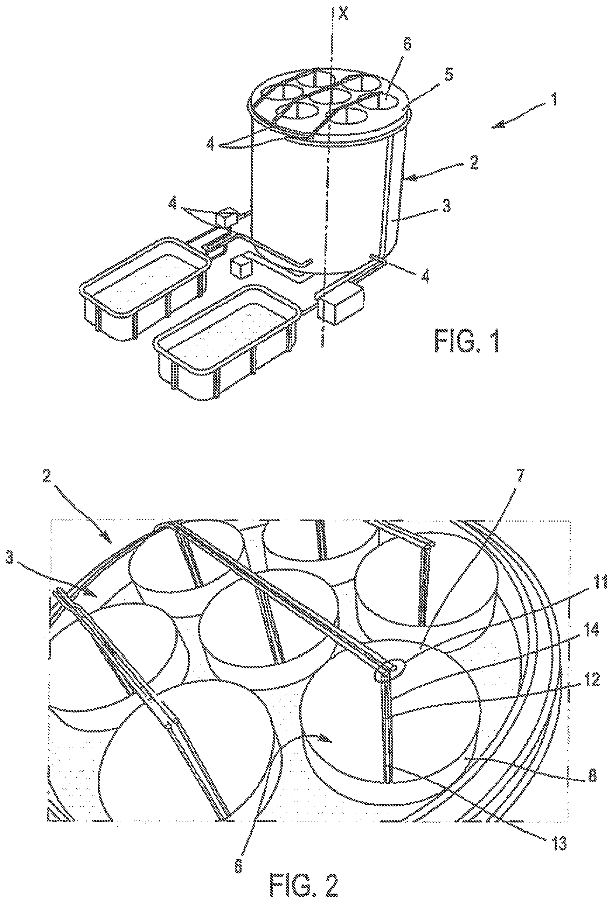

La figure 1 illustre un mode de réalisation de l'invention dans lequel, le dispositif de production d'une culture photosynthétique 1 comprend au moins un photo-bioréacteur 2 formant une enceinte 3 munie de moyens d'alimentation/évacuation 4, et apte à contenir une eau algale.

A titre d'exemple, l'enceinte peut être une cavité naturelle tel un lac, un étang, ou dans la mer ou artificielle, en béton, en tôle, en géo-membrane, etc. Elle peut être peinte en blanc permettant ainsi la réflexion de la lumière, enduite d'une matière étanche à l'eau, et/ou isolée thermiquement de l'intérieur et/ou de l'extérieur.

Ledit photo-bioréacteur peut être de forme cubique, parallélépipédique, cylindrique, sphérique, pyramidale, prismatique ou toutes variantes de ceux-ci. De préférence, on choisira une forme parallélépipédique ou cylindrique dont les dimensions sont adaptées à la production industrielle d'une culture photosynthétique. A titre d'exemple pour un photo-bioréacteur parallélépipédique : 100m sur 30m et 8m de hauteur ; ou pour un petit photo-bioréacteur cylindrique : 7m de diamètre sur 4m de hauteur. Bien sûr, les dimensions du photo-bioréacteur seront à déterminer et adapter selon le type d'installation industrielle souhaitée.

Préférentiellement, ledit photo-bioréacteur est hors sol ; ou afin de limiter l'encombrement spatial d'une telle installation, il peut être en partie enterré. Il peut être placé à proximité d'une usine émettrice de CO2, d'une station de traitement des eaux usées, de méthaniseurs / digesteurs, dans le cadre de valorisations (Biogaz, cogénération, compostage, recyclage du CO2). Par ailleurs, il peut être placé sur des terres arides ou dans des déserts, à proximité de la mer pour l'apport en eau.

Ladite enceinte 3 est recouverte à son extrémité proximale, par un moyen de recouvrement 5, suffisamment résistant afin d'au moins tolérer le poids d'un homme une fois installé. Par exemple une bâche plastique ou une combinaison de couches de polymères, ou bien encore une toile imperméable de type polyester avec une enduction PVC typiquement utilisée pour la couverture de tentes ou des remorques des poids-lourds.

L'épaisseur du moyen de recouvrement 5 est par exemple de 0,6mm avec un poids d'environ 600g/m$^2$. En outre, le moyen de recouvrement 5 possède à sa périphérie un moyen d'attache 21 pour permettre une application étanche sur les parois de l'enceinte 3. Cela peut-être par exemple un ourlet avec des oeillets dans lesquels passent des tendeurs, ou bien des fourreaux dans lesquels des tubes métalliques sont insérés. En outre, il peut être avantageux que sa densité soit inférieure à 1 pour qu'elle puisse flotter afin de faciliter son installation.

Ledit photo-bioréacteur 2 est muni d'au moins un distributeur de lumière 6.

Ledit au moins un distributeur de lumière 6 est de préférence de forme tubulaire, cylindrique, s'étendant longitudinalement selon un axe X, dont les dimensions sont adaptées aux dimensions du photo-bioréacteur.

La forme tubulaire du distributeur de lumière 6 a été retenue car elle est simple et facilement réalisable en usine, même pour des grandes dimensions, par rapport à d'autres formes géométriques.

Ledit au moins un distributeur de lumière ou « ballon » 6 est délimité par une membrane souple de préférence en polymère, apte à faire varier le volume dudit au moins un distributeur de lumière 6 tel un ballon gonflable/dégonflable et possède au moins deux parois, au moins une première paroi 7 agencée pour recevoir de la lumière à une extrémité proximale, au moins une seconde paroi 8 agencée pour émettre au moins une partie de la lumière reçue.

La membrane souple est de préférence faiblement extensible et peu élastique, afin de minimiser le phénomène de ballonnement sur la partie tubulaire du ballon 6. L'épaisseur de la membrane peut être par exemple environ 200$\mu$m. La membrane permettant la réalisation de la cavité étanche du distributeur de lumière 6 peut être constituée de plusieurs parties soudées entre elles. Par exemple, une longue partie tubulaire avec deux extrémités soudées. Les nombreux films transparents utilisés notamment pour les serres, sont très économiques car fabriqués en grande quantité par méthode d'extrusion gonflage ou de co-extrusion. Ces procédés de fabrication sont très avantageux, car non seulement les coûts de production sont les plus faibles, mais ils permettent de constituer des films multicouches associant différentes caractéristiques. L'homme de l'art saura parfaitement utiliser ces procédés connus pour fabriquer le film transparent adapté à la constitution des parois 7 et 8 du distributeur de lumière 6.

En outre, le degré de transmission lumineuse globale de la membrane 7,8 est de préférence supérieur à 90%. L'effet diffusant du polymère doit altérer le plus faiblement possible son degré de transmission lumineuse. La transmission de lumière est une propriété importante de la membrane. Néanmoins, il faut bien distinguer la différence entre la lumière directe et la lumière indirecte aussi appelée diffuse. Trop de lumière directe pourrait entraîner une destruction des micro-algues, et des pertes par absorption sous forme calorique, surtout dans les régions à fort rayonnement solaire. L'incorporation de charges minérales dans la fabrication de la membrane permet d'obtenir une meilleure répartition de la lumière qui devient plus diffuse et plus homogène. En outre, des co-polymères (type EVA) ont pour fonction de bloquer les radiations des infrarouges longs, et ralentissent les baisses de températures.

La paroi 7 réceptrice de lumière en partie proximale du distributeur de lumière est de préférence de forme légèrement bombée. Cette forme permet une meilleure captation de la lumière par rapport à la position du soleil tout le long de la journée.

Les dimensions, le nombre de distributeurs de lumière 6 ainsi que la distance entre chaque distributeur de lumière 6 sont déterminés à partir des formules suivantes

$$nb_{distributeur} = \frac{4.\xi.S_{enceinte}}{\pi.D^2} \quad (1)$$

$$H_{distributeur} = \frac{D}{4.q_2}(\eta.q_{solaire} - q_2) \quad (2)$$

$$d = \sqrt{\frac{\pi.D^2}{4.\xi.\cos 30}} \quad (3)$$

Avec :

$H_{distributeur}$ : hauteur de la seconde paroi 8 du distributeur de lumière 6 (parie immergée) (en m)

**D** : le diamètre du distributeur de lumière 6 (en m).

$q_{solaire}$ : flux incident d'une journée ensoleillée (en $\mu$moles de photons/m$^2$.s$^{-1}$)

$q_2$ : flux lumineux souhaité à la paroi émettrice de lumière du distributeur de lumière pour la bonne production d'une souche de micro-algue donnée en ($\mu$mol/m$^2$.s$^{-1}$).

$\eta$ : le rendement de transmission entre la première paroi 7 réceptrice de lumière et la seconde paroi 8 émettrice du distributeur de lumière.

$nb_{distributeur}$ : le nombre de disributeur de lumière 6 dans l'enceinte 3 du photobioréacteur

$S_{enceinte}$ : surface d'une enceinte de section transversale circulaire ou rectangulaire (en m$^2$

$\xi$ : la fraction volumique occupée par les distributeurs de lumière, rapportée au volume total d'eau dans l'enceinte.

La fraction $\xi$ est de préférence comprise entre 0,6 à 0,8.

**d** : distance en mètre entre les axes longitudinaux X de 2 distributeurs de lumière.

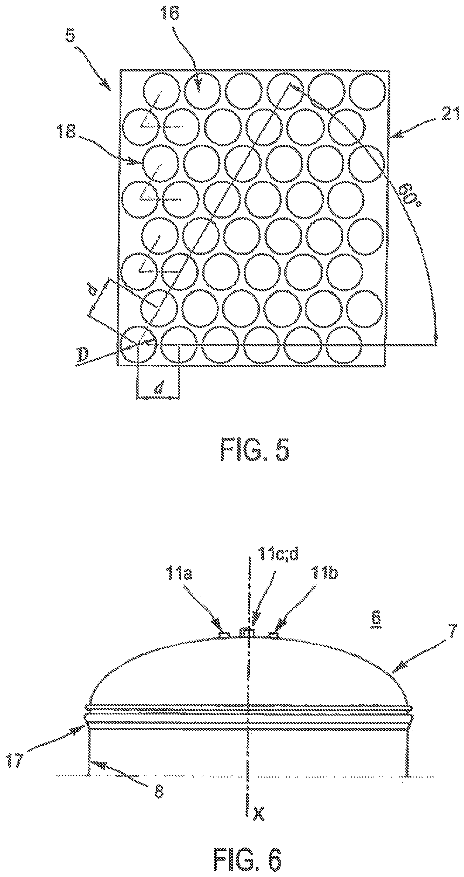

A titre illustratif, pour une fraction epsilon $\xi$ égale à 0,6857, et lorsque le diamètre **D** de chaque distributeur vaut 2 mètres, la distance **d** entre les axes longitudinaux X de 2 distributeurs de lumière directement voisins est 2,30m, soit 30cm d'espace entre les parois tel que l'illustre la figure 5.

La distance **d** peut être comprise entre par exemple 1m et 3m.

La valeur epsilon $\xi$ représente également en partie proximale, la surface couverte par la totalité desdits distributeurs de lumière par rapport à la surface totale du photo-bioréacteur 2. A par exemple = 0,667, les 2/3 de la surface de l'enceinte 3 est couverte par la première paroi 7 du distributeur de lumière 6, le dernier tiers étant couvert par le moyen de recouvrement 5. Pour une fraction $\xi$ comprise entre 0,6 et 0,8, l'eau contenue dans les distributeurs de lumière représente donc environ 60% à 80% du volume total d'eau dans l'enceinte 3, et l'eau algale représentera 20% à 40% du volume d'eau total.

Bien entendu, les dimensions mentionnées dans la présente demande du photo-bioréacteur 2 et du au moins un distributeur de lumière 6 ne sont pas exhaustives et pourront évoluer en fonction des performances obtenues et de l'ergonomie recherchée.

Le nombre de distributeurs de lumière pourra par exemple être compris entre 1 et 10000 par enceinte 3. Les distributeurs de lumière 6 sont disposés de façons équidistantes les uns des autres dans l'enceinte 3 tel que le montre la figure 5. Cette disposition forme une succession de rangées placées en quinconce, c'est-à-dire qu'une rangée est décalée de la demi distance entre deux distributeurs de lumière, par rapport à la rangée précédente et la suivante. Cette disposition optimise la surface lumineuse utile dans le volume d'eau algale.

L'avantage de l'introduction de distributeurs de lumière dans l'eau algale est prioritairement de gérer la grande quantité de flux de lumière en excès du soleil. Dans un bassin de type « raceway », lors d'une journée ensoleillée, la quantité de lumière arrive directement sur un plan d'eau horizontal. Si une partie de la quantité de photons est absorbée par photosynthèse, une grande partie de l'énergie lumineuse sera perdue sous forme de chaleur. En effet, les micro-algues jusqu'à un certain seuil n'absorbent plus une luminosité trop importante, et inversement cela peut générer de la photo-inhibition. En outre, un excès de la température de l'eau en surface peut inhiber la croissance des micro-algues et beaucoup d'eau est perdue par évaporation. Une approche en trois dimensions permet de contourner ces problèmes, en diffusant le flux lumineux sur une superficie maximale de manière à augmenter la quantité de photons captés par les micro-algues. Aussi, la pénétration de la lumière jusqu'à la partie distale du ballon 6 est déterminante pour la bonne production photosynthétique, d'où l'intérêt d'une membrane aux caractéristiques appropriées permettant de capter au mieux le flux lumineux du soleil, et de le diffuser de la façon la plus homogène possible sur toute la surface de la seconde paroi 8 émettrice de lumière.

La quantité de lumière $q_{solaire}$ varie selon la latitude géographique du lieu d'implantation du site de production. L'éclairement d'une journée ensoleillée d'été en France est de 80 000 Lux, soit un flux incident $q_{solaire}$ d'environ 1350 $\mu mol/m^2.s^{-1}$. Les micro-algues n'ont besoin que de quelques $\mu$moles de photons/$m^2.s^{-1}$ voire quelques dizaines de $\mu$moles/$m^2.s^{-1}$ pour dépasser le point de compensation entre respiration et photosynthèse, et ainsi commencer à croître et à se dupliquer. Le tableau ci-dessous reprend les résultats de différentes études publiées sur la production journalière de micro-algues diverses, suivant l'intensité du flux lumineux :

| Souche micro-algue | Concentration cellulaire en phase stationnaire (x10E6 cell/ml) | Production volumique biomasse (g/L/jr) | Production lipide (g/L/jr) | Flux lumineux ($\mu mol/m^2/s$) | Références littérature |
|---|---|---|---|---|---|
| Chlorella Vulgaris | 6 | 0,037 | 40% | 76 | Illman et al. (2000) |
| Chlorella Vulgaris | | 0,485 | | | Dragone et al. (2011) |
| Chlorella Vulgaris | | 0,254 | | | Liang et al. (2009) |
| Chlorella Vulgaris | | 1,437 | | 400 | Shih-Hsin Ho et al. (2012) |
| Chlorella Vulgaris | | 0,39 | | 90 | Shih-Hsin Ho et al. (2012) |
| Chlorella Vulgaris | | 0,272 | 0,144 | | Yeh and Chang (2012) |
| Chlorella Vulgaris | 33,2 | | | 100 | Jean Hee Bae et al. (2011) |
| Chlorella Vulgaris | 64,9 | | | 100 | Jean Hee Bae et al. (2011) |
| Chlorella Vulgaris | 227,2 | | | | Zaleha & al. (2013) |
| Nannochloropsis oculata | 112,3 | | | 100 | Jean Hee Bae et al. (2011) |
| Nannochloropsis oculata | | 0,382 | | 0,17 | Su et al. (2011) |
| Nannochloropsis oculata | | | 0,151 | | Chiu et al. (2009) |
| Nannochloropsis oculata | *(7,9g/L)* | 1,5 | | 21 | Ramanathan & al. (2011) |
| Nannochloropsis sp. | 22,5 | | | 34 | Roncallo & Al. (2012) |

(suite)

| Souche micro-algue | Concentration cellulaire en phase stationnaire (x10E6 cell/ml) | Production volumique biomasse (g/L/jr) | Production lipide (g/L/jr) | Flux lumineux ($\mu$mol/m$^2$/s) | Références littérature |
|---|---|---|---|---|---|
| Nannochloropsis sp. | 37,5 | | | 34 | Roncallo & Al. (2012) |
| Nannochloropsis sp. | | 0,124 | 0,074 | | Jiang et al. (2011) |
| Nannochloropsis sp. | | | 0,148 | | Cheirsilp and Torpee (2012) |
| Nannochloropsis sp. | 107,3 | | | 100 | Jean Hee Bae et al. (2011) |
| Nannochloropsis sp. | 92,2 | | | 17 *8h/24* | Zaleha & al. (2013) |
| Desmodesmus sp. | | 0,762 | 0,263 | 700 | Shih-Hsin Ho et al. (2014) |
| Desmodesmus sp. | | 0,433 | 0,213 | 700 | Shih-Hsin Ho et al. (2014) |
| Desmodesmus sp. | | 0,67 | 0,302 | 700 | Shih-Hsin Ho et al. (2014) |
| Scenedesmus obliquus | | 0,151 | | 60 | Shih-Hsin Ho et al. (2010) |
| Scenedesmus obliquus | | 0,201 | | 60 | Shih-Hsin Ho et al. (2010) |
| Nannochlorum sp. | 250 | | | 80 | Pereira et al. (2011) |
| Tetraselmis sp. | 40 | | | 80 | Pereira et al. (2011) |
| Tetraselmis sp. | *(12,38g/L)* | | 0,173 | 30 *12h/24* | Mohd Shamzi & al. (2013) |
| Chaetoceros calcitrans | 20 | | | 52 | Robert & His (1987) |
| Chaetoceros calcitrans | 14,7 | | 35,40% | | Kwangdinata & al. (2014) |
| Chaetoceros calcitrans | *(8,1g/L)* | 1,7 | | 21 | Ramanathan & al. (2011) |
| Chaetoceros calcitrans | 10 | | | 13 | Kaspar & al. (2013) |
| Chlamydomonas sp | *7,5 (4g/L)* | | 0,169 | 250 | Nakanishi & al. (2013) |
| Spirulina Platensis | *(1,59g/L)* | | | 52 | Chauhan & Pathak (2010) |
| Spirulina Platensis | *(1,84g/L)* | | | 77 *12h/24* | Gami & al. (2011) |

Bien que les études soient très souvent réalisées sur de petits volumes en laboratoire, la disparité des performances de production pour un éclairement donné est grande. Certaines études montrent cependant de bonnes productions volumiques avec un éclairement faible de quelques dizaines de μmoles de photons/m$^2$.s$^{-1}$ seulement. L'une des études de Roncallo & al. (2012) donne une concentration maximale de micro-algues *Nannochloropsis sp.* de 37,5x10$^6$ cellules/ml pour une luminosité d'environ 34 μmol/m$^2$.s$^{-1}$ (2000 Lux) seulement, dans un photo-bioréacteur à colonne verticale de 25 cm de diamètre. L'une des études de Ramanathan & al. (2011) montre une production journalière de *Chaetoceros Calcitrans* jusqu'à 1,7 g/L.jour$^{-1}$, pour une luminosité de seulement 21 μmol/m$^2$.s$^{-1}$ (1200 Lux), dans des tubes verticaux de 4,5 cm de diamètre.

Dans la littérature actuelle, les rendements en production surfacique en g/m$^2$.jour$^{-1}$ sont disparates. Les résultats vont de quelques grammes par m$^2$.jour$^{-1}$ à 130 grammes par m$^2$.jour$^{-1}$. L'étude indienne de Sudhakar & al. (2012) montre par exemple des rendements surfaciques en bassin conventionnel type raceway en moyenne de 73 g/m$^2$.jour$^{-1}$ et 76 g/m$^2$.jour$^{-1}$ sur deux sites de production de biomasse distincts.

En reprenant l'équation (2) avec les valeurs *D* et *q$_{solaire}$* sus mentionnées en exemple, en partant sur une quantité de lumière *q$_2$* émise à la surface de la seconde paroi 8 par exemple 85 μmol/m$^2$.s suffisante pour la bonne croissance d'une souche de micro-algue donnée, et d'un rendement de transmission $\eta$ à 80% dans la cavité étanche du ballon 6, la hauteur *H* de la seconde paroi 8 du distributeur de lumière vaudra alors 6m.

| Flux lumineux q2 émis dans le distributeur avec D=2m H=6m rdt=80% | | | |
|---|---|---|---|
| | Ciel couvert | Beau temps | |
| Eclairement : | 25000 | 80000 | Lux |
| Flux incident q solaire : | 430 | 1377 | μmol/m$^2$/s |
| q2 distributeur : | 26 | 85 | μmol/m$^2$/s |

L'absorption du rayonnement visible dans la hauteur de colonne d'eau du distributeur de lumière 6 devra être la plus faible afin de garder un bon rendement $\eta$ de transmission de la lumière. C'est-à-dire d'avoir une eau filtrée avec la plus faible turbidité. Dans l'eau, la lumière rouge dans la gamme de longueurs d'ondes située entre 600 à 700 μm est plus rapidement absorbée que la lumière bleue (400 à 500 μm). A 10m de profondeur, environ 80% de la lumière bleue est encore disponible dans une eau claire, alors que le rouge sera entièrement absorbé. Toutefois, toutes les micro-algues photo-synthétisent entre autre la lumière bleue. L'homme de l'art en tiendra évidement compte, en fonction de la hauteur d'eau voulue dans l'enceinte 3. Par exemple, chez les Diatomées, la production de lipides est favorisée par les longueurs d'ondes bleues, justement celles qui sont les moins rapidement absorbées par l'eau. L'eau contenue dans la cavité étanche du ballon 6 peut-être de l'eau douce ou de l'eau de mer. La plupart des substances composant le sel marin ont peu d'effet sur l'absorption de la lumière dans l'eau. Il n'y a pratiquement pas de différence entre le spectre d'absorption d'une eau de mer très claire et celui de l'eau distillée.

Une quantité de photons maximale de 300 μmol/m$^2$.s$^{-1}$ pourra être distribuée sur la surface de la seconde paroi 8 du ballon 6. Pour une quantité de PAR (Rayonnement Photosynthétiquement Actif, exprimé en μmol/m$^2$.s$^{-1}$ ou en W/m$^2$) donnée à une latitude géographique, la quantité moyenne de photons disponible dans l'enceinte 3 dépend évidemment du diamètre du distributeur de lumière proportionnellement à sa profondeur, cette quantité est calculée suivant la formule (2).

En outre, la concentration maximale des micro-algues dans l'eau algale est à adapter pour une production photosynthétique quotidienne optimale. Le mode de production en semi-continu est intéressant. Il consiste à concentrer la densité cellulaire jusqu'à la phase stationnaire, c'est-à-dire le seuil auquel les micro-algues ne peuvent plus se développer davantage dans un milieu de culture et à une luminosité donnés. A ce seuil de saturation, un prélèvement d'une partie de l'eau algale est réalisé par le moyen d'évacuation 4, pour être filtré et en récolter la biomasse. L'étude de Shih-Hsin Ho & al. (2014) montre qu'un prélèvement à 90% du volume d'eau algale tous les 5 jours permet une meilleure production volumique journalière de micro-algues *Desmodesmus sp.* qu'un prélèvement à 10% tous les deux jours (0,67 g/L/jr contre 0,20 g/L/jr respectivement). La fréquence de la récolte dependra de la rapidité de croissance de la souche de micro-algue donnée, pour arriver au seuil de saturation, et de la quantité d'eau algale prélevée à chaque récolte.

Une production de bio-carburant peut être calculée en se basant sur une productivité volumique théorique, chiffrée dans le tableau suivant :

| Nombre de jours de production : | 300 | jours/an |
|---|---|---|
| Diamètre D des distributeurs 6 : | 2 | m |
| Hauteur H des distributeurs 6 : | 6 | m |

(suite)

| | | |
|---|---|---|
| Surface émettrice des distributeurs : | 40,86 | m² |
| Valeur fraction Epsilon : | 0,7495 | |
| Distance d entre distributeurs (aux axes X) : | 2,2 | m |
| Nombre de distributeurs /ha (équation 2) : | 2386 | |
| Nombre de m² surfacique par hectare : | 97490 | m²/ha |
| Volume d'eau algale : | 16539 | m³/ha |
| Equivalence profondeur raceway : | 17 | cm |
| Production surfacique dans distributeur : | 60 | g/m²/j |
| Production volumique biomasse : | 0,354 | g/L/j |
| % de terrain non occupé (chemins, etc) : | 15% | par ha |
| Production biomasse à l'hectare : | 1492 | T/ha/an |
| % de lipides dans la biomasse : | 30% | |
| Production annuelle Bio-carburant à l'ha : | 508000 | L/ha/an |

Dans un mode de réalisation alternatif, le diamètre de la première paroi 7 peut être différent du diamètre de la seconde paroi 8.

Dans une autre réalisation alternative de l'invention, la première paroi 7 recevant la lumière est en plastique rigide de type polycarbonate, et la seconde paroi 8 de type membrane souple. L'interface entre les deux parois 7, 8 est étanche. En outre, les deux parois peuvent être séparées pour le stockage et/ou la maintenance.

Par ailleurs, selon un autre mode de réalisation, ledit ballon 6 est muni à son extrémité proximale d'un anneau de levage ou tout autre moyen techniquement équivalent apte à extraire ledit ballon (de préférence sous sa forme dégonflée), de ladite enceinte 3.

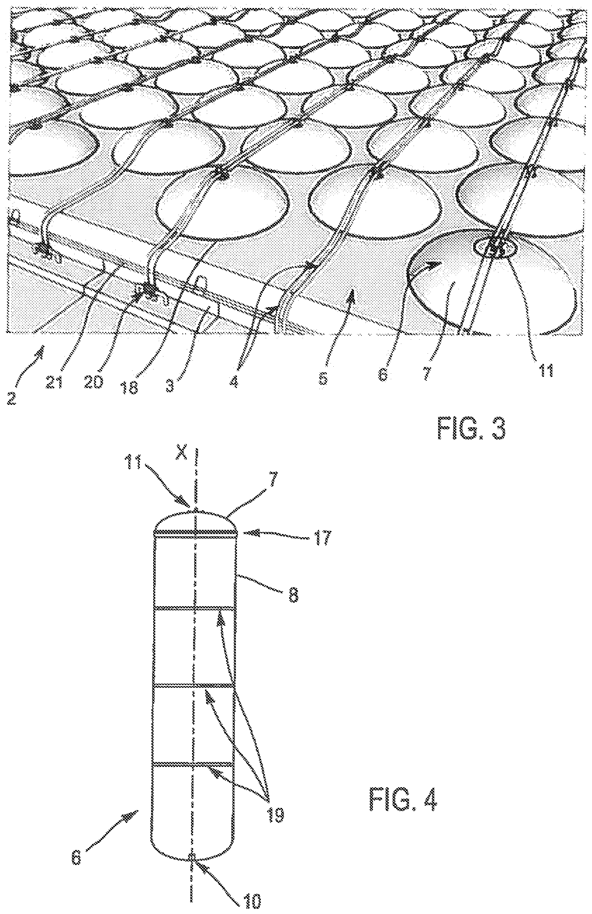

En outre, ledit ballon 6 est muni à son extrémité proximale de moyens 11 indépendants d'alimentation et de refoulement, apte à remplir et à extraire un ou des fluides de la cavité étanche délimitée par les parois 7 et 8, tel que le montre la figure 3. Ces moyens peuvent être des inserts soudés à la paroi 7 du ballon 6. Ces inserts comprennent un taraudage intérieur pour permettre le branchement aux circuits, et peuvent être de tailles différentes suivant le type de fluide à faire circuler.

Dans un mode de réalisation préféré, lesdits fluides introduits dans la cavité étanche sont de l'eau et de l'air, aptes à contrôler l'immersion dudit au moins un distributeur de lumière 6 dans l'eau algale. L'eau contenue dans la cavité étanche est la plus claire possible et filtrée, pour une bonne transmission lumineuse en surface comme en profondeur.

Dans un autre mode de réalisation, ledit au moins un distributeur de lumière 6 possède deux cavités étanches séparées par une membrane transparente, une pour l'air en partie proximale, une pour l'eau pour l'immersion en partie distale.

En outre, de façon à maintenir immergé ledit au moins un distributeur de lumière 6 selon un plan parallèle à l'axe X, à la manière d'un « bouchon de pèche », ledit au moins un distributeur de lumière 6 est muni sur son extrémité distale d'une masse 10. Ladite masse 10 peut être en métal, en béton, ou une capsule en plastique moulé remplie de sable. La forme de la masse est de préférence plate, de manière à prendre un minimum d'encombrement selon l'axe orthogonal X.

Selon des modes de réalisation alternatifs, ladite masse 10 peut : être vissée dans un insert, pendre le long d'un lien situé à l'extrémité distale dudit au moins un ballon 6, ou de préférence se trouver à l'extrémité d'un guide 9 traversant de part en part ledit ballon 6, ou tout autre moyen techniquement équivalent.

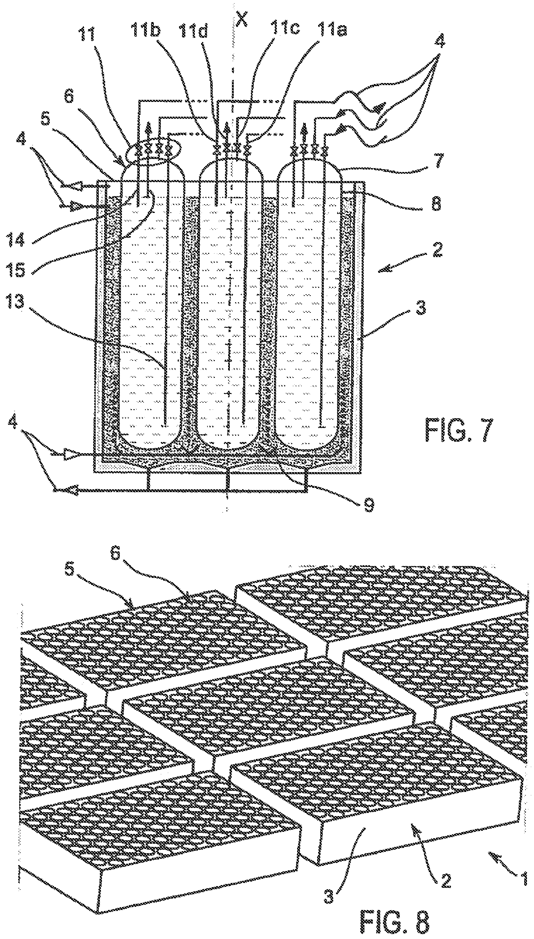

Dans un mode de réalisation préféré, lesdits moyens 11 indépendants d'alimentation et de refoulement sont constitués d'un moyen d'alimentation en eau 11a, d'un moyen d'évacuation (ou de refoulement) en eau 11b, d'un moyen d'alimentation en air 11c et d'un moyen d'échappement d'air 11d, ces moyens 11 peuvent être constitués de vannes, soupapes ou tout autre moyen connu de l'homme du métier. Les figures 6 et 7 illustrent cet arrangement. Ainsi, un tel arrangement peut permettre d'isoler du circuit d'alimentation et de refoulement un distributeur 6 spécifique pendant une phase de maintenance sans compromettre les autres distributeurs de lumière 6 dudit dispositif de production 1.

En outre comme illustré sur les figures 2 et 7, ledit moyen d'alimentation en eau 11a est muni d'un tuyau dit long 13 qui s'étend sensiblement selon l'axe longitudinal X et débouche dans le fond dudit ballon ; ledit moyen d'évacuation (ou de refoulement) en eau 11b est muni d'un tuyau dit court 14 qui débouche en dessous du plan formé par l'interface entre l'eau et l'air, par exemple à 10 ou 20cm dudit plan ; ledit moyen d'échappement d'air 11d est muni d'un tuyau d'évacuation d'air 15 qui débouche sur le plan formant l'interface entre et l'eau et l'air. Ce tuyau d'évacuation d'air 15 est apte à établir la profondeur d'immersion du ballon 6 dans l'eau algale en contrôlant le volume d'air dans le ballon 6. De préférence, lesdits tuyaux court 14, long 13 et ledit tuyau d'évacuation d'air 15 sont attachés audit guide 12 ; en outre, ledit moyen d'alimentation en air 11c, et ledit moyen d'échappement d'air 11d peuvent être confondus tel que le montrent les figures 2, 4 et 6.

Les moyens d'alimentation et d'évacuation en air 11c et 11d sont des moyens physiques simples pour absorber la dilatation ou la contraction de l'air à l'intérieur de la cavité étanche en partie proximale du distributeur de lumière 6, quel que soit la fluctuation de la température météorologique quotidienne. Ainsi le volume d'air en partie proximale reste constant, assurant la bonne immersion dudit ballon 6 dans l'eau algale.

Par ailleurs, le tuyau long 13 d'alimentation peut également être utilisé pour vidanger le ballon 6. Dans ce cas, il sera percé préférentiellement régulièrement sur toute sa longueur pour mieux vidanger ledit ballon 6 et ainsi éviter un colmatage trop rapide entre les parois dudit ballon et le tuyau 13. Une autre solution est que ledit tuyau 13 est muni d'une crépine à son extrémité.

Optionnellement, le tuyau d'évacuation de l'air peut avoir des longueurs différentes afin d'obtenir un décalage de hauteur entre par exemple deux rangées de ballons 6 dans le photo-bioréacteur 2. Cela peut être particulièrement intéressant pour créer une légère pente du moyen de recouvrement 5, et faciliter ainsi l'évacuation des eaux de pluies. Ainsi le moyen de recouvrement peut être par exemple plus éloigné de la partie distale au milieu du photo-bioréacteur que des côtés. Le dénivelé pourra être léger, de préférence inférieur à 2%.

Optionnellement, un niveau pour mesurer la hauteur de l'eau contenue dans la cavité étanche du distributeur de lumière 6 est relié à un boîtier électronique externe de gestion (non représenté). Ce niveau peut être une sonde fixée ou insérée sur le guide 12 ou bien sur le tuyau long 13.

Les branchements desdits moyens 11 d'alimentation et de refoulement sur le moyen d'évacuation 4 coopèrent avec le moyen de gestion via une rampe 10 reliant plusieurs distributeurs de lumière 6, par exemple quatre ballons. Puis au bord de l'enceinte, la rampe est branchée au moyen de gestion rejoignant par exemple un local de pompage et de filtration de l'eau et de l'air des ballons. Pour une enceinte de grande dimension, chaque rangée de ballons est par exemple alimentée par plusieurs rampes branchées bout à bout via des tuyaux souples entre-elles. Aussi pour faciliter la maintenance de manière localisée, des vannes 20 sont placées aux extrémités des rangées de ballons, et accessibles depuis le bord de l'enceinte, afin d'isoler la rangée en question du reste du circuit.

Optionnellement comme illustré sur la figure 4, ledit au moins un distributeur de lumière 6 peut être pourvu d'au moins un moyen 19 de maintien additionnel permettant de rigidifier ses parois (garder l'aspect tubulaire du distributeur de lumière 6) et d'éviter un effet bombé sur la seconde paroi. Ces moyens 19 peuvent être des cerclages espacés sur la hauteur du distributeur de lumière 6 ou une surépaisseur localisée de la seconde paroi.

Comme illustré sur la figure 7, ledit photo-bioréacteur comprend au moins un moyen de bullage 9 d'un gaz de préférence de l'air, en particulier avec apport additionnel en $CO_2$ (avec éventuellement un apport en nutriments), disposé au moins sur une partie du fond de l'enceinte 3 du photo-bioréacteur 2, de façon à permettre un brassage permanent dudit liquide aqueux comprenant ladite culture photosynthétique et d'évacuer l'oxygène produit par la photosynthèse hors de l'eau algale, permettant ainsi d'éviter le risque d'une forte sursaturation en oxygène et par conséquent une inhibition de croissance des micro-algues. Pour ce faire, la vitesse d'ascension des bulles pourra de préférence être comprise entre 0,3 à 0,35m/s.

En outre, de préférence, ledit au moins un moyen de bullage 9 n'est pas placé à l'aplomb dudit au moins un distributeur de lumière 6 ; il est placé par exemple entre deux distributeurs de lumière 6.

Par ailleurs, l'accroissement du nombre de moyens de bullage 9 au $m^2$ a pour avantage d'éviter les phénomènes de « spiral-flows ». Ce phénomène de courant ascendant de l'eau algale créé par l'ascension des bulles qui accélère la vitesse de passage de celles-ci, et affecte l'échange gazeux. Le rapprochement des moyens de bullage 9 entre eux casse le risque de « spiral-flows ».

L'air diffusé dans l'eau algale peut contenir une concentration en $CO_2$ plus importante que celle de l'air atmosphérique (400ppm), dans le but de diminuer le besoin en aération et son coût. Par exemple une proportion du $CO_2$ dans l'air apporté à 1000ppm serait suffisante pour diviser par deux environ, les besoins et coûts en aération, sans affecter la bonne évacuation de l'oxygène. La proximité du dispositif de production 1 avec une usine émettrice de $CO_2$, ou bien l'apport en $CO_2$ depuis des digesteurs de biomasse intégrés au dispositif de production seraient un choix judicieux pour optimiser les coûts de production.

Le moyen de bullage 9 peut comprendre des diffuseurs à membrane en EPDM permettant une aération en fines bulles. Il se présente sous forme de disques fixés au réseau d'apport en air. Les diffuseurs d'air standard utilisés pour l'aération des bassins des stations d'épuration peuvent convenir tant qu'ils sont adaptés à la pression de colonne d'eau dudit photo-bioréacteur.

En outre, dans un mode de réalisation alternatif, un moyen de brassage mécanique peut être utilisé en addition dudit moyen de bullage 9 par exemple avec des hélices.

Optionnellement, une ou plusieurs lampes LED peuvent être placées à l'intérieur de la cavité étanche du distributeur de lumière 6, par exemple le long du guide 12, et reliés électriquement. De telles lampes LED permettent d'augmenter la production photosynthétique quotidienne des micro-algues. Les lampes LED sont de préférence immergées dans l'eau du ballon 6 pour une meilleure diffusion lumineuse. Elles pourront avoir une puissance de plusieurs watts et émettre préférentiellement dans la gamme de longueur d'onde d'absorption de la chlorophylle lors de la photosynthèse (400nm - 700nm). Cet éclairage pourra compléter l'éclairage naturel les jours de faible ensoleillement, et/ou fonctionner la nuit, sachant qu'il pourra être interrompu suivant des séquences en rapport aux cycles biologiques des micro-algues.

Comme illustré sur les figures 3 et 5, le moyen de recouvrement 5 de ladite extrémité proximale de l'enceinte 3 du photo-bioréacteur 2 est munie d'au moins une ouverture 16 apte à recevoir en son sein et à maintenir ledit au moins un distributeur de lumière 6 dans une position fixe dans ladite enceinte du photo- bioréacteur 2. Ainsi lorsque cela est nécessaire, un distributeur de lumière 6 peut être remplacé sans avoir à toucher audit moyen de recouvrement, ni aux autres distributeurs de lumière 6.

Comme montré sur la figure 6 représentant un mode de réalisation alternatif, le maintien dudit au moins un distributeur de lumière 6 dans ladite au moins une ouverture 16 se fait par coopération de formes mécaniques entre un relief de surface 17 dudit au moins un distributeur de lumière 6 et un bord 18 de ladite ouverture 16.

Ledit relief de surface 17 peut être un relief négatif dans la paroi dudit au moins un distributeur de lumière 6 tel qu'une rainure ou gorge dans laquelle vient se loger ledit bord 18, ou une série de reliefs positifs tel un rail ou un bourrelet encadrant ledit bord 18, tel que le montre la figure 6.

Dans un mode de réalisation alternatif, ledit relief de surface 17 peut être un fourreau intermédiaire soudé entre les parois 7 et 8. Une ou plusieurs bandes flexibles ou profilés semi-rigides peuvent être insérées dans le fourreau afin de former le bourrelet ou un anneau précontraint prêt à être logé dans ladite ouverture 16.

En outre, le relief de surface 17 peut être un fourreau gonflable indépendamment de la cavité étanche du distributeur de lumière 6.

De façon intéressante, lesdits fluides au sein dudit au moins un ballon 6 créent une surpression, déformant les parois dudit au moins un distributeur de lumière. Ceci permet de créer une coopération étanche entre la paroi dudit au moins un distributeur de lumière 6 et le bord 18 de l'ouverture 16 du moyen de recouvrement ou bâche 5.

Dans un mode autre de réalisation alternatif, ledit au moins un distributeur de lumière 6 possède un diamètre légèrement supérieur à celui de ladite au moins une ouverture 16 ; ainsi lorsque ledit au moins un distributeur de lumière est gonflé celui-ci est coincé par l'ouverture 16 lors de la dite coopération de forme.

Sous la bâche 5, l'évacuation de l'air saturé en eau (ou éventuellement d'éthanol) et de l'oxygène, se fait par des moyens d'évacuation 4 tel que des tuyères placées le long de ladite enceinte 3 sans pression excessive, pour éviter les fuites. Ces gaz sont éventuellement acheminés vers un local pour en récupérer l'eau, les calories et l'oxygène.

Ainsi, l'eau évaporée peut s'écouler à travers un condenseur permettant de diminuer drastiquement l'apport en eau durant le fonctionnement du photo-bioréacteur. Un équipement adapté peut condenser plus de 90% de l'eau évaporée, et ainsi la recycler dans l'eau algale de l'enceinte 3. Cet arrangeant procédé peut en outre, être associé à un échangeur de calories air /air ou air / eau, ou une pompe à chaleur car l'air sortant de l'enceinte 3, plus chaud et saturé, peut facilement chauffer l'eau alimentant les ballons 6, ou l'air chargé en $CO_2$ diffusé dans l'eau algale.

Selon un mode de réalisation alternatif, les moyens d'évacuation 4 peuvent être connectés directement sur la bâche 5.

Selon l'invention, des moyens d'alimentation/évacuation 4 tel que des bouches de refoulement en eau algale filtrée sont situées sur les parois de l'enceinte 3 du photo-bioréacteur, notamment vers l'extrémité proximale sous le plan d'eau algale pour permettre un remplissage sans turbulence de l'enceinte vis-à-vis dudit au moins un distributeur de lumière 6. En outre, la vidange de l'eau algale peut se faire par un réseau d'évacuation quadrillant le fond de ladite enceinte 3, par exemple, sous chaque distributeur de lumière 6. L'eau algale peut également rejoindre un bassin de stockage ou une usine de traitement et de filtration, de centrifugation et de transformation de la biomasse, via des collecteurs appropriés.

Tous les collecteurs d'alimentation et de retour pour l'eau algale, l'eau et l'air des distributeurs de lumière, et l'air servant au bullage, peuvent longer plusieurs photo-bioréacteurs, être fixés en hauteur le long des parois extérieurs de l'enceinte 3, être enterrés, être insérés entre deux parois murales séparant deux photo-bioréacteurs, ou encore faire partie d'un ensemble structuré séparant deux photo-bioréacteurs.

En outre, l'enceinte du photo-bioréacteur est équipée de sondes (niveau, température, composition, pH, salinité, turbidité, $O_2$, $CO_2$, etc.), de compteurs et d'automatismes de contrôle intégrés dans un système de gestion.

Dans un mode particulier de l'invention, les nutriments servant au développement du milieu de culture sont injectés dans l'eau algale après la filtration, c'est-à-dire sur un circuit retournant à l'enceinte 3.

Afin d'obtenir une installation de taille industrielle, ledit dispositif de production 1 peut comprendre une pluralité de photo-bioréacteurs 2 dont les enceintes 3 sont installées côte à côte comme l'illustre la figure 8. Dans ce cas, les moyens d'alimentation, d'extraction, de refoulement, d'évacuation ainsi que les moyens de contrôle et de gestion et les moyens de stockage, peuvent être mutualisés entre les différents photo-bioréacteurs ou groupes de photo- bioréacteurs.

De façon intéressante, le moyen de recouvrement 5 et la paroi 7 des distributeurs de lumière 6 permettent d'obtenir un effet de serre entre ceux-ci et le plan d'eau algale. Le principe de la serre est un moyen simple pour capter l'énergie solaire en excès non utilisée par la photosynthèse des micro-algues. Récupérée sous forme de chaleur, il contribue à la montée générale de la température du milieu de culture.

L'opportunité d'utiliser un photo-bioréacteur de plusieurs mètres de profondeur avec un grand volume d'eau présente plusieurs avantages. Tout d'abord la stabilité de la température du milieu de culture. Les micro-algues sont très sensibles aux variations de températures. L'étude américaine de James & Boriah (2010) montre qu'à une profondeur de 60cm, la production de biomasse est deux fois plus importante qu'à 10cm de profondeur, même avec une luminosité plus faible, du fait de la meilleure stabilité de la température. L'inertie générée par le volume d'eau est donc très importante pour contrer les oscillations de températures atmosphériques quotidiennes.

En outre, le volume d'eau contenu dans l'enceinte 3 permet de dissiper la chaleur captée par cet effet de serre en partie proximale, évitant ainsi les excès de températures dû au fort ensoleillement en surface. La hauteur de colonne d'eau sert en quelque sorte d'accumulateur de calories. Ainsi, l'énergie lumineuse fournie au $m^2$ par le soleil est davantage exploitée par le photo- bioréacteur, améliorant son rendement en prolongeant la saison de récolte. En effet cette accumulation de chaleur n'est pas perdue et sert au développement des micro-algues les jours de mauvais temps. L'analogie peut être faite à celle d'une culture maraîchère sous serre et une culture de plein champ : la culture sous serre permet une production quasi permanente alors qu'elle n'est que de quelques mois en plein champ. Autre analogie possible, la température de l'eau d'une piscine sous couvrant en plexiglas reste plus élevée et pendant plus longtemps dans la saison en comparaison d'une piscine non couverte.

Aussi la profondeur de l'enceinte 3 sera à adapter en fonction de l'emplacement géographique du photo-bioréacteur, la quantité d'énergie solaire variant suivant la latitude terrestre. Par exemple un bassin peu profond au niveau de l'équateur aura une température d'eau algale trop élevée pour la croissance photosynthétique, car la dissipation de la chaleur sera trop faible.

D'autre part, le moyen de recouvrement 5 et la paroi 7 des distributeurs de lumière 6 ont une fonction d'isolation aux intempéries, de par la couche d'air qui les sépare du plan d'eau. Ainsi la température du milieu de culture en partie proximale sera moins influencée par les conditions météorologiques quotidiennes (vents froids, vents chauds, fortes pluies, etc).

Le brassage de l'eau algale par l'aération de fines bulles permet l'homogénéisation de la température sur toute la hauteur de colonne d'eau. Aussi, la circulation de l'eau à l'intérieure de la cavité étanche des distributeurs de lumière 6 est importante pour homogénéiser la température de haut en bas dans l'enceinte 3. La circulation lente et continue de l'eau de surface chauffée par le soleil vers la partie distale du distributeur de lumière s'effectue par les moyens 11 indépendants d'alimentation et de refoulement.

En outre, la température de l'eau de la cavité étanche peut être régulée par un apport ou un retrait en calories extérieures, par exemple un échangeur de calories ou tout autre moyen de chauffage ou refroidissement. Ainsi, tel un « bain marie » inversé, la température régulée de chaque distributeur de lumière vient à se dissiper et réguler à son tour la température de l'eau algale dans l'enceinte 3.

Mise en oeuvre d'un dispositif selon l'invention

Une méthode de mise en oeuvre consiste à faire glisser la bâche 5 au-dessus de l'enceinte 3 pleine d'eau, en y insérant les ballons 6 au fur et à mesure depuis le bord de l'enceinte 3. La bâche 5 est tendue et maintenue à la surface de l'eau. Des ballons 6 sont placés dans une première rangée d'ouvertures 16 de la bâche 5, et sont branchés entre eux. De l'air est injecté pour permettre leur flottaison dans l'eau de l'enceinte 3. Ainsi de suite pour les autres rangées, tout en faisant avancer la bâche 5 pour couvrir enfin l'enceinte 3. La liaison entre la bâche 5 et l'enceinte 3 est rendue étanche. De l'eau est ajoutée dans les ballons 6 jusqu'à obtention de la mise en pression. Bien que l'étanchéité soit déjà existante à partir de ce moment entre ledit ballon 6 et l'ouverture 16, le bord 18 de celle-ci n'est pas emboîté dans le relief de surface 17 dudit ballon 6. Pour cela, de l'air est injecté sous la bâche 5, alors que le ratio air/eau dans les ballons 6 diminue. La pression sous la bâche 5 fait monter le bord 18 de l'ouverture 16 le long de la paroi 7 dudit ballon 6 jusqu'à son emboîtement dans le relief 17.

Selon une autre méthode de mise en oeuvre, la bâche 5 peut être installée au-dessus de l'enceinte 3 préalablement remplie d'eau, à l'aide d'une grue ou d'un portique mobile. Lesdits ballons 6 sont placés à travers les ouvertures 16 à l'aide d'une grue ou d'une passerelle motorisée spécialement développée à cette tâche. Lesdits ballons 6 sont déployés, branchés, remplis d'eau et d'air avec un ratio air/eau faible. La membrane dudit ballon 6 est alors tendue par la mise en pression. Le ratio air/eau dans lesdits ballons 6 est augmenté de manière à ce qu'ils soulèvent la bâche 5. L'ensemble fait que la bâche 5 est maintenant suspendue par lesdits ballons 6. L'emboîtement entre le bord 18 de ladite ouverture 16 et le relief de surface 17 dudit ballon 6 peut s'effectuer grâce à une force exercée vers le bas sur la bâche 6 à l'aide d'un outillage adapté à cet effet. La hauteur de flottaison de chaque ballon 6 peut être régulée en insufflant de l'air jusqu'à ce qu'il soit refoulé par un tuyau 15 prévu à cet effet. Le niveau de l'eau algale et celui des ballons 6 peuvent être ajusté de façon à obtenir une hauteur de bâche 5 légèrement supérieure au bord du mur de l'enceinte 3, pour faciliter l'écoulement des eaux de pluies vers la périphérie du photo-bioréacteur.

**Revendications**

1. Dispositif de production (1) d'une culture photosynthétique comprenant au moins un photo-bioréacteur (2) formant une enceinte (3) munie de moyens d'alimentation/évacuation (4) et comprenant :

   - un liquide aqueux comprenant une culture photosynthétique,
   - au moins un moyen (4) d'alimentation et d'évacuation en fluides de ladite enceinte coopérant avec un système de gestion,
   - au moins un distributeur de lumière (6) comprenant :

     - au moins une première paroi (7) agencée pour recevoir de la lumière à une extrémité proximale,
     - au moins une seconde paroi (8) agencée pour émettre au moins une partie de la lumière reçue,
     - une cavité étanche délimitée par lesdites au moins une première (7) et une seconde parois (8),
     - au moins une partie de la paroi émettrice (8) est immergée dans le liquide aqueux comprenant la culture photosynthétique,
     - au moins un fluide remplissant au moins en partie ladite cavité étanche,

     - un moyen de recouvrement (5) dudit photo-bioréacteur (2) apte à limiter l'évaporation dudit liquide aqueux,

   lequel moyen de recouvrement (5) est muni d'au moins une ouverture (16), apte à maintenir ledit au moins un distributeur de lumière (6) dans une position fixe dans ladite enceinte (3) du photo-bioréacteur (2),
   **caractérisé en ce que** ledit au moins un distributeur de lumière est flottant et permet de soutenir le moyen de recouvrement au-dessus du plan d'eau algale.

2. Dispositif de production d'une culture photosynthétique selon la revendication 1, **caractérisé en ce que** ledit au moins un distributeur de lumière permet de soutenir le moyen de recouvrement au-dessus du plan d'eau algale à une distance comprise entre 10 et 50 cm.

3. Dispositif de production d'une culture photosynthétique selon l'une quelconque des revendications 1 ou 2, **caractérisé en ce qu'**une coopération de forme entre au moins une bordure (18) de ladite ouverture dudit moyen de recouvrement (5) et un relief de surface (17) dudit au moins un distributeur de lumière (6) est réalisée, afin de maintenir ledit au moins un distributeur de lumière (6) dans une position fixe dans ladite enceinte (3) du photo-bioréacteur (2).

4. Dispositif de production d'une culture photosynthétique selon l'une des revendications 1 à 3, caractérisé en que ladite paroi (7) de réception de lumière dudit au moins un distributeur de lumière est disposée à l'extérieur du volume défini par ladite enceinte (3) du photo-bioréacteur(2).

5. Dispositif de production d'une culture photosynthétique selon l'une des revendications 1 à 4, **caractérisé en ce que** le nombre ($nb_{distributeur}$) et la hauteur immergée ($H_{distributeur}$) dudit au moins un distributeur de lumière (6) et l'espacement (d) entre les différents distributeurs de lumière (6) sont déterminés à partir des formules suivantes :

$$nb_{distributeur} = \frac{4.\xi.S_{ence\,int\,e}}{\pi.D^2} \qquad (1)$$

   $\xi$: la fraction volumique occupée par les distributeurs de lumière (6), rapportée au volume total d'eau dans l'enceinte (3).
   **D** : le diamètre du distributeur de lumière (6) (en m).
   $S_{enceinte}$: la surface d'une enceinte (3) de section transversale circulaire ou rectangulaire (en $m^2$).

$$H_{distributeur} = \frac{D}{4.q_2}(\eta.q_{solaire} - q_2) \qquad (2)$$

$H_{distributeur}$: hauteur de la seconde paroi (8) du distributeur de lumière (partie immergée) (en m).

$q_{solaire}$: flux incident d'une journée ensoleillée (en $\mu$moles de photons /$m^2.s^{-1}$).

$q_2$ : flux lumineux souhaité à la paroi émettrice de lumière du distributeur de lumière pour la bonne production d'une souche de micro-algue donnée (en $\mu$mol/$m^2.s^{-1}$).

$\eta$ : le rendement de transmission entre la première paroi (7) réceptrice de lumière et la seconde paroi (8) émettrice du distributeur de lumière

$$d = \sqrt{\frac{\pi.D^2}{4.\xi.\cos 30}} \qquad (3)$$

$d$: distance entre les axes longitudinaux X de 2 distributeurs de lumière (6) (en m).

6. Dispositif de production d'une culture photosynthétique selon l'une des revendications 1 à 5, **caractérisé en ce que** ledit au moins un distributeur de lumière (6) est muni de moyens (11, 11a, 11b, 11c, 11d) d'alimentation et d'extraction en fluide.

7. Dispositif de production d'une culture photosynthétique selon l'une des revendications 1 à 6, **caractérisé en ce que** le fluide remplissant au moins en partie ladite cavité étanche est composé d'eau et d'air, apte à mettre en pression le volume intérieur dudit au moins un distributeur de lumière (6) de façon à rendre étanche la coopération de forme entre la bordure (18) de ladite ouverture (16) et ledit relief de surface (17) dudit au moins un distributeur de lumière (6).

8. Dispositif de production d'une culture photosynthétique selon l'une des revendications 1 à 7, **caractérisé en ce que** ledit au moins un distributeur de lumière (6) est muni d'un moyen d'ancrage (10) disposé à une extrémité distale.

9. Dispositif de production d'une culture photosynthétique selon l'une des revendications 1 à 8, **caractérisé en ce que** lesdites parois (7,8) dudit au moins un distributeur de lumière sont formées par au moins une membrane souple en polymère.

10. Dispositif de production d'une culture photosynthétique selon la revendication précédente, **caractérisé en ce que** ladite membrane souple a reçu ou comprend lors de sa fabrication au moins un traitement de surface intérieur et/ou extérieur parmi les suivants : anti-goutte, anti-poussière, filtre UV et/ou infrarouges, stabilisants UV, effet diffusant, hydrophobe, anti-poussière, anti- algue.

11. Dispositif de production d'une culture photosynthétique selon l'une des revendications 1 à 10, **caractérisé en ce que** ledit photo-bioréacteur (2) comprend au moins un moyen de bullage (9) avec apport en $CO_2$, disposé au moins sur une partie du fond de l'enceinte (3) du photo-bioréacteur (2), de façon à permettre un brassage permanent dudit liquide aqueux comprenant ladite culture photosynthétique.

**Patentansprüche**

1. Vorrichtung (1) zur Herstellung einer photosynthetischen Kultur mit wenigstens einem Lichtbioreaktor (2), der ein mit Zuleitungs- und Ableitungsmitteln (4) versehenes Gehäuse (3) bildet, und mit

   - einer eine photosynthetische Kultur beinhaltenden wässrigen Flüssigkeit,
   - wenigstens einem Mittel (4) zum Zuleiten und Ableiten von Fluiden des Gehäuses, die mit einem Steuerungssystem zusammenwirken,
   - wenigstens einem Lichtverteiler (6), der

- wenigstens eine erste Wand (7), die dazu ausgelegt ist, Licht an einem proximalen Ende aufzunehmen,
- wenigstens eine zweite Wand (8), die dazu ausgelegt ist, wenigstens einen Teil des aufgenommenen Lichts abzustrahlen,
- eine dichte Kammer, die durch die wenigstens eine erste (7) und die wenigstens eine zweite (8) Wand begrenzt ist,
- wobei wenigstens ein Teil der abstrahlenden Wand (8) in die die photosynthetische Kultur beinhaltende wässrige Flüssigkeit eingetaucht ist,
- wenigstens ein die dichte Kammer wenigstens teilweise füllendes Fluid

aufweist,
- einem Mittel (5) zum Abdecken des Lichtbioreaktors (2), das dazu ausgelegt ist, das Verdunsten der wässrigen Flüssigkeit zu begrenzen,

wobei das Abdeckmittel (5) mit wenigstens einer Öffnung (16) versehen ist, die dazu ausgelegt ist, den wenigstens einen Lichtverteiler (6) im Gehäuse (3) des Lichtbioreaktors (2) in einer festen Position zu halten, **dadurch gekennzeichnet, daß** der wenigstens eine Lichtverteiler schwimmt und ermöglicht, das Abdeckmittel oberhalb der Algenwasserebene zu halten.

2. Vorrichtung zur Herstellung einer photosynthetischen Kultur gemäß Anspruch 1, **dadurch gekennzeichnet, daß** der wenigstens eine Lichtverteiler ermöglicht, das Abdeckmittel in einem Abstand von 10 bis 50 cm oberhalb der Algenwasserebene zu halten,

3. Vorrichtung zur Herstellung einer photosynthetischen Kultur gemäß einem der Ansprüche 1 oder 2, **dadurch gekennzeichnet, daß** ein Formschluß zwischen wenigstens einem Rand (18) der Öffnung des Abdeckmittels (5) und einem Oberflächenrelief (17) des wenigstens einen Lichtverteilers (6) gebildet ist, um den wenigstens einen Lichtverteiler (6) in einer festen Position im Gehäuse (3) des Lichtbioreaktors (2) zu halten.

4. Vorrichtung zur Herstellung einer photosynthetischen Kultur gemäß einem der Ansprüche 1 bis 3, **dadurch gekennzeichnet, daß** die Licht aufnehmende Wand (7) des wenigstens einen Lichtverteilers außerhalb des durch das Gehäuse (3) des Lichtbioreaktors (2) definierten Volumens angeordnet ist.

5. Vorrichtung zur Herstellung einer photosynthetischen Kultur gemäß einem der Ansprüche 1 bis 4, **dadurch gekennzeichnet, daß** die Anzahl ($nb_{Verteiler}$) und die Eintauchtiefe ($H_{Verteiler}$) des wenigstens einen Lichtverteilers (6) und der Abstand zwischen den verschiedenen Lichtverteilern (6) durch die folgenden Formeln bestimmt sind:

$$nb_{Verteiler} = \frac{4 \cdot \xi \cdot S_{Gehäuse}}{\pi \cdot D^2} \qquad (1)$$

$\xi$ : der von den Lichtverteilern (6) besetzte Volumenanteil, bezogen auf das Gesamtvolumen des Wassers im Gehäuse (3);
D : der Durchmesser des Lichtverteilers (6) (in m);
$S_{Gehäuse}$ : die Fläche eines Gehäuses (3) mit kreisförmigem oder rechteckigem Querschnitt (in $m^2$).

$$H_{Verteiler} = \frac{D}{4 \cdot q_2} (\eta \cdot q_{Sonne} - q_2) \qquad (2)$$

$H_{Verteiler}$ : Höhe der zweiten Wand (8) des Lichtverteilers (eingetauchter Teil) (in m);
$q_{Sonne}$ : einfallender Lichtfluß eines sonnigen Tages (in $\mu$mol Photone/$m^2 \cdot s^{-1}$);
$q_2$ : gewünschter Lichtfluß an der Licht abstrahlenden Wand des Lichtverteilers für die gute Produktion eines gegebenen Mikroalgenstamms (in $\mu$mol/$m^2 \cdot s^{-1}$);
$\eta$ : Wirkungsgrad der Übertragung zwischen der Licht aufnehmenden ersten Wand (7) und der Licht abstrahlenden zweiten Wand (8) des Lichtverteilers.

$$d = \sqrt{\frac{\pi \cdot D^2}{4 \cdot \xi \cdot \cos 30}} \qquad (3)$$

d : Entfernung zwischen den Längsachsen X zweier Lichtverteiler (6) (in m).

**6.** Vorrichtung zur Herstellung einer photosynthetischen Kultur gemäß einem der Ansprüche 1 bis 5, **dadurch gekennzeichnet, daß** der wenigstens eine Lichtverteiler (6) mit Mitteln (11, 11a, 11b, 11c, 11d) zum Zuleiten und Ableiten von Fluid versehen ist.

**7.** Vorrichtung zur Herstellung einer photosynthetischen Kultur gemäß einem der Ansprüche 1 bis 6, **dadurch gekennzeichnet, daß** das die dichte Kammer wenigstens teilweise füllende Fluid aus Wasser und Luft zusammengesetzt ist und geeignet ist, das innere Volumen des wenigstens einen Lichtverteilers (6) unter Druck zu setzen, um den Formschluß zwischen dem Rand (18) der Öffnung (16) und dem Oberflächenrelief (17) des wenigstens einen Lichtverteilers (6) abzudichten.

**8.** Vorrichtung zur Herstellung einer photosynthetischen Kultur gemäß einem der Ansprüche 1 bis 7, **dadurch gekennzeichnet, daß** der wenigstens eine Lichtverteiler (6) mit einem an einem distalen Ende angeordneten Verankerungsmittel (10) versehen ist.

**9.** Vorrichtung zur Herstellung einer photosynthetischen Kultur gemäß einem der Ansprüche 1 bis 8, **dadurch gekennzeichnet, daß** die Wände (7, 8) des wenigstens einen Lichtverteilers durch wenigstens eine flexible Polymermembrane gebildet sind.

**10.** Vorrichtung zur Herstellung einer photosynthetischen Kultur gemäß dem vorangehenden Anspruch, **dadurch gekennzeichnet, daß** die flexible Membrane während ihrer Herstellung wenigstens eine innere und/oder äußere Oberflächenbehandlung unter den folgenden erhalten hat oder aufweist: gegen Tropfen, gegen Staub, UV- und/oder Infrarotfilter, UV-Stabilisator, Streueffekt, wasserabweisend, gegen Staub, gegen Algen.

**11.** Vorrichtung zur Herstellung einer photosynthetischen Kultur gemäß einem der Ansprüche 1 bis 10, **dadurch gekennzeichnet, daß** der Lichtbioreaktor (2) wenigstens ein Mittel (9) zur Blasenbildung mit $CO_2$-Zufuhr aufweist, das wenigstens auf einem Teil des Bodens des Gehäuses (3) des Lichtbioreaktors (2) angeordnet ist, um ein ständiges Umrühren der die photosynthetische Kultur enthaltenden wässrigen Flüssigkeit zu ermöglichen.

**Claims**

**1.** Device for producing (1) a photosynthetic culture comprising at least one photo-bioreactor (2) forming a chamber (3) having a supply/discharge means (4) and comprising:

- an aqueous liquid comprising a photosynthetic culture,
- at least one means (4) for supplying and for discharging fluids from said chamber interacting with a management system,
- at least one light distributor (6) comprising:

- at least one first wall (7) arranged so as to receive the light at a proximal end,
- at least one second wall (8) arranged so as to emit at least one part of the received light,
- a sealed cavity defined by said at least one first (7) and one second walls (8),
- at least one part of the emitting wall (8) is immersed in the aqueous liquid comprising the photosynthetic culture,
- at least one fluid, at least partially filling said sealed cavity,

- a covering means (5) of said photo-bioreactor (2) capable of limiting the evaporation of said aqueous liquid,

wherein said covering means (5) has at least one opening (16), capable of keeping said at least one light distributor (6) in a stationary position in said chamber (3) of the photo-bioreactor (2),

**characterised in that** said at least one light distributor is floating and makes it possible to support the covering means above the plane of algal water.

2. Device for producing a photosynthetic culture according to claim 1, **characterised in that** said at least one light distributor makes it possible to support the covering means above the plane of algal water at a distance between 10 and 50 cm.

3. Device for producing a photosynthetic culture according to any of the claims 1 or 2, **characterised in that** a cooperation of form between at least one edge (18) of said opening of said covering means (5) and a surface relief (17) of said at least one light distributor (6) is carried out, in order to maintain said at least one light distributor (6) in a stationary position in said chamber (3) of the photo-bioreactor (2).

4. Device for producing a photosynthetic culture according to one of claims 1 to 3, **characterised in that** said wall (7) for receiving light of said at least one light distributor is arranged outside of the volume defined by said chamber (3) of the photo-bioreactor (2).

5. Device for producing a photosynthetic culture according to one of claims 1 to 4, **characterised in that** the number ($nb_{distributor}$) and the immersed height ($H_{distributor}$) of said at least one light distributor (6) and the spacing (d) between the different light distributors (6) are determined using the following formulas:

$$nb_{distributeur} = \frac{4.\xi.S_{enceinte}}{\pi.D^2} \quad (1)$$

distributeur = distributor

$\xi$: the volume fraction occupied by the light distributors (6), compared to the total volume of water in the chamber (3).
D: the diameter of the light distributor (6) (in m).
$S_{chamber}$: the surface of a chamber (3) of circular or rectangular transversal section (in $m^2$).

$$H_{distributeur} = \frac{D}{4.q_2}(\eta.q_{solaire} - q_2) \quad (2)$$

distributeur = distributor
solaire = solar

$H_{distributor}$: height of the second wall (8) of the light distributor (immersed portion) (in m).
$q_{solar}$: incident flux of a sunny day (in $\mu$moles of photons/$m^2$.$s^{-1}$).
$q_2$: luminous flux desired at the light emitting wall of the light distributor for the good production of a given strain of micro-algae (in $\mu$mole/$m^2$.$s^{-1}$).
$\eta$: the transmission output between the first light receiving wall (7) and the second emitting wall (8) of the light distributor

$$d = \sqrt{\frac{\pi.D^2}{4.\xi.\cos 30}} \quad (3)$$

d: distance between the longitudinal axes X of 2 light distributors (6) (in m).

6. Device for producing a photosynthetic culture according to one of claims 1 to 5, **characterised in that** said at least one light distributor (6) is provided with means (11, 11a, 11b, 11c, 11d) for supplying and for extracting fluid.

7. Device for producing a photosynthetic culture according to one of claims 1 to 6, **characterised in that** the fluid, at least partially filling said sealed cavity, is comprised of water and of air, able to pressurise the interior volume of said at least one light distributor (6) in such a way as to render the cooperation of form watertight between the edge (18) of said opening (16) and said surface relief (17) of said at least one light distributor (6).

8. Device for producing a photosynthetic culture according to one of claims 1 to 7, **characterised in that** said at least one light distributor (6) is provided with a means of anchoring (10) arranged at a distal end.

9. Device for producing a photosynthetic culture according to one of claims 1 to 8, **characterised in that** said walls (7,8) of said at least one light distributor are formed by at least one flexible membrane made of polymer.

10. Device for producing a photosynthetic culture as claimed in the preceding claim, **characterised in that** said flexible membrane received or comprises during its manufacture at least one internal and/or external surface treatment from among the following: anti-drip, anti-dust, UV and/or infrared filter, UV stabilisers, diffusing effect, hydrophobic, anti-dust, anti-algae.

11. Device for producing a photosynthetic culture according to one of claims 1 to 10, **characterised in that** said photo-bioreactor (2) comprises at least one means for bubbling (9) with a supply of $CO_2$, arranged at least on a portion of the bottom of the chamber (3) of the photo-bioreactor (2), in such a way as to allow for a permanent stirring of the aqueous liquid comprising said photosynthetic culture.

FIG. 1

FIG. 2

21

FIG. 3

FIG. 4

FIG. 5

FIG. 6

FIG. 7

FIG. 8

**EP 3 227 427 B1**

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- WO 2013063075 A
- WO 2012152637 A
- WO 2009116853 A
- WO 2013011448 A